# EUROPEAN PATENT APPLICATION

(11) **EP 4 085 946 A1**
(43) Date of publication of application: **09.11.2022**
(21) Application number: 21171891.1
(22) Date of filing: 03.05.2021
(51) Int. Cl.: A61M 5/315, A61M 5/20, A61M 5/24

(54) **METHOD FOR IDENTIFYING A DRUG DELIVERY DEVICE, SET OF A DRUG DELIVERY DEVICE AND AN ELECTRONIC DEVICE FOR IDENTIFYING A DRUG DELIVERY DEVICE**

(71) Applicant: medmix Switzerland AG, 9469 Haag (Rheintal) (CH)
(72) Inventor: Gabriel, Frédéric, 8052 Zürich (CH); Lurf, Robert, 8051 Zürich (CH)
(74) Representative: Manitz Finsterwald Patent- und Rechtsanwaltspartnerschaft mbB

(57) **Abstract**

The present invention relates to a method for identifying a drug delivery device, such as a pen-injector, using an electronic device. The invention further relates to a set of a drug delivery device and an electronic device, and to an electronic device.

## Description

The present invention relates to a method for identifying a drug delivery device, such as a pen-injector, using an electronic device. The invention further relates to a set of a drug delivery device and an electronic device, and to an electronic device.

Market and stakeholder analyses show the need to support and guide patients during the usage of injection devices to increase therapy adherence. Therefore, the knowledge of the dialled and/or expelled dose are important parameters.

To acquire the remaining dose in the injection pen, so called connected caps exist that are based on a bundle of infrared emitters corresponding sensors. However, such caps do not include an implemented and dedicated identification technology.

In this regard add on devices often use dedicated identification technologies like NFC (near field communication), RFID (radio frequency identification), QR-codes, barcodes or similar technologies to identify the injector device by its automated readable unique ID.

Thus, besides the components required for the dose measurement additional components are needed realizing the pen identification.

The subsequent integration of such a technology would lead to a massive redesign of the system, larger dimensions of the cap and additional costs for implementation of the new sensors.

For this reason it is an object of the invention to make available an as compact as possible system and method for detecting both an amount of medicament present in an injection device which also permits a reliable detection of the kind of detection device storing the medicament. It is a further object of the invention to make available an as cost effective as possible system and method that work reliably.

This object is satisfied by a method for identifying a drug delivery device, such as a pen-injector, using an electronic device, wherein the electronic device comprises a housing having a cavity, a sensor assembly, and a processing unit,
wherein the sensor assembly and the processing unit are arranged inside the housing,
wherein the drug delivery device comprises a drug compartment for holding a liquid to be delivered by the drug delivery device and an identifier,
wherein the sensor assembly comprises an emitter unit and a detector unit for generating detector signals, wherein the method comprises: receiving the drug compartment and the identifier inside the cavity of the housing of the electronic device; emitting electromagnetic radiation from the emitter unit into the cavity; receiving at least part of the electromagnetic radiation from the cavity with the detector unit and generating a detector signal that represents the electromagnetic radiation received by the detector unit; determining from the detector signal a volume of the liquid inside the drug compartment using the processing unit; detecting a presence of the identifier from the detector signal using the processing unit; and distinguishing, using the processing unit, the drug delivery device based on the identifier from a further drug delivery device having a further identifier that is different from the identifier, with the identifier being provided on, for example printed on or glued to, a surface of the drug delivery device.

The invention thus makes available a feasible approach to sense the expelled dose without impacting the mechanics or function of the injection pen. This is achieved by providing a separate, i.e. additional, electronic devices that measures an identifier present on a surface, preferably an outer surface of the drug delivery device. Such an electronic device is combined with well-known connected caps. This reduces the complexity and size of the electronic device in a favorable manner.

The emitter unit can e.g. comprise a dedicated number of infrared emitters and a slightly higher numbers of infrared receivers can be aligned opposite of the emitter unit as the detection unit within the electronic device, preferably within the connected cap. When the drug delivery device is inserted into the connected cap a certain infrared shadow is acquired by the receivers. This shadow pattern is aligned with the value of the remaining drug volume present in the drug delivery device.
The detection of different versions of a drug delivery device by one single electronic device, e.g. cap is crucial to avoid mixing up different versions of a medicament or drug delivery device during a course of therapy. If a user were to mix up the different medicaments this would lead to reduced adherence, in worst cases to higher severity risks to patients. Furthermore, such detection would help to optimize the usability of the electronic device, e.g. cap, allowing the use of one cap for several types of drug delivery devices in contrast to using one electronic device for each kind of drug delivery device.

The present invention thus uses the already available infrared sensors within an electronic device, which are originally used to acquire only the remaining dose in the drug delivery device. The use of this infrared system also allows an identification of the inserted drug delivery device or of foreign objects.

The processing unit may determine the volume of the liquid from a first signal component of the detector signal and may detect the identifier from a second signal component of the detector signal different from the first signal component, wherein, for example, the signal components amount to signals generated by spatially separated sensor elements of the detection unit. In this way an as compact as possible electronic device and hence an as efficient as possible method of identifying can be implemented as there is no need to completely redesign the electronic device.

The identifier may be located at a distal end of the drug compartment facing away from a delivery end of the drug delivery device. The positioning of the identifier at such a distal position means that the identification of the identifier can be conducted without interfering with the detection of the volume of medicament in the drug delivery device.

The identifier may be located at a longitudinal position that radially overlaps with the drug compartment. In this way an as compact as possible assembly is made available.

The identifier may comprise a first and second portion, wherein the first and second portion are spaced apart from each other along a longitudinal axis of the drug delivery device.

A distance between the first and second portion may be larger than a longitudinal extent of a movable plunger sealing the drug compartment. Using two portions as the identifier increases the number of codes available for detection by the electronic device and hence permits a larger number of differentiations between different kinds of drug delivery devices respectively of medicament stored therein.

The identifier may comprise a pattern with a modulated refractive index at a wavelength of the electromagnetic radiation. Such patterns can be printed in a simple manner on the drug delivery device. Moreover, such patterns allow an improved identification of the identifier of the drug delivery device.

The pattern may provide a modulated attenuation of the electromagnetic radiation, wherein the detector signal may represent a shadow cast by the identifier on the detector unit. Such patterns can be printed in a simple manner on the drug delivery device. Moreover, such patterns allow an improved identification of the identifier of the drug delivery device.

One of the identifier and the pattern may comprise a circumferential marker that surrounds a longitudinal axis of the drug delivery device. Such circumferential markers can be identified in a simple manner independent of the positioning of the electronic device relative to the drug delivery device. The different axial positions of such circumferential positions for different types of pens or pens of the same kind having different medicament therein permits a differentiation therebetween.

One of the identifier and the pattern may comprise a longitudinal marker that only covers a limited radial sector around a longitudinal axis of the drug delivery device. Such longitudinal markers allow the identification of different drug delivery devices based on the radial position of the marker about the longitudinal axis.

The identifier may be a coding feature for a specific a drug delivery device, wherein the method comprises the step of decoding the coding feature to determine the type of a drug delivery device inserted into the cavity of the electronic device. The use of such coding features allows a drug delivery device specific identification to take place by means of the method presented herein.

The coding feature may comprise a member selected from the group of members consisting of one or more straight lines of different size and/or colour, one or more curved lines of different size and/or colour, one or more zigzag lines of different size and/or colour, one or more dashed lines of different size and/or colour, fluorescent markings, a colored shape, a transparent code that filters only part of the spectrum of the electromagnet radiation; and combinations of the foregoing. Such coding features are simple to manufacture in a cost effective and expedient manner even in existing manufacturing plants.

The identifier may be provided at a cartridge holder of the drug delivery device, wherein the cartridge holder may be configured to receive a cartridge that comprises the drug compartment or may be integrally formed with the cartridge. Such an arrangement permits an as compact as possible assembly of the electronic device to be realized.

The electronic device may comprise a memory that stores at least two different sets of configuration data, each set of configuration data being associated with a different type of drug delivery device and/or liquid,
wherein the method may further comprise:
- selecting a selected set from the sets of configuration data using the processing unit based on the identifier detected by the processing unit.

In this way the electronic device can store data for different types of pen and medicament and one can thereby ensure an as precise a detection of the volume of the medicament stored in the respective drug delivery device.

According to a further aspect the above object is also satisfied by a set of a drug delivery device, such as an injection pen, and an electronic device for attachment to the drug delivery device,
wherein the drug delivery device may comprise a drug compartment for holding a liquid to be delivered by the drug delivery device and an identifier provided on a surface of the drug delivery device,
wherein the electronic device may comprise a housing, a sensor assembly and a processing unit,
wherein the sensor assembly and the processing unit are arranged inside the housing, wherein the housing comprises a cavity that is configured to receive the drug compartment and the identifier of the drug delivery device,
wherein the sensor assembly comprises an emitter unit and a detector unit,
wherein the emitter unit is configured to emit electromagnetic radiation into the cavity, wherein the detector unit is configured to receive at least part of the electromagnetic radiation from the cavity and to generate a detector signal that represents the electromagnetic radiation received by the detector unit,
wherein the processing unit is configured to determine from the detector signal a volume of the liquid inside the drug compartment,
wherein the processing unit is further configured to detect a presence of the identifier from the detector signal and to distinguish the drug delivery device based on the identifier from a further drug delivery device having a further identifier that is different from the identifier.

In this way an as simple as possible and as compact as possible set comprising a possibly hitherto generally known drug delivery device and electronic device is made available. The advantages discussed in the foregoing in connection with the method likewise hold true for the set.

According to yet a further aspect the above object is also satisfied by an electronic device for attachment to a drug delivery device, such as an injection pen,
wherein the electronic device comprises a housing, a sensor assembly and a processing unit,
wherein the sensor assembly and the processing unit are arranged inside the housing, wherein the housing comprises a cavity that is configured to receive a drug compartment for holding a liquid to be delivered by the drug delivery device and an identifier of the drug delivery device that is provided on a surface of the drug delivery device,
wherein the sensor assembly comprises an emitter unit and a detector unit,
wherein the emitter unit is configured to emit electromagnetic radiation into the cavity, wherein the detector unit is configured to receive at least part of the electromagnetic radiation from the cavity and to generate a detector signal that represents the electromagnetic radiation received by the detector unit,
wherein the processing unit is configured to determine from the detector signal a volume of the liquid inside the compartment,
wherein the processing unit is further configured to detect a presence of the identifier from the detector signal and to distinguish the drug delivery device based on the identifier from a further drug delivery device having a further identifier that is different from the identifier.

In this way an as simple as possible and as compact as possible electronic device is made available. The advantages discussed in the foregoing in connection with the method likewise hold true for the electronic device.

In the following detailed description of the present disclosure, reference will be made to the accompanying drawings, of which
- Fig. 1: is a perspective illustration of a disposable drug delivery device;
- Fig. 2: shows a perspective illustration of the device of Fig. 1 where the cap is removed allowing attachment of a pen needle to the cartridge holder;
- Fig. 3: is an exploded view of the device of Fig. 1;
- Fig. 4: a perspective view of a reusable drug delivery device with an attached cap;
- Fig. 5: a perspective view of the drug delivery device of Fig. 4 with the cap removed and an attached dispensing unit;
- Fig. 6: a perspective view of the drug delivery device of Fig. 4, the cap and the dispensing unit;
- Fig. 7: a side view of the dispensing unit of Fig. 6 comprising a cartridge holder and a cartridge and a pen needle attachable to the dispensing unit;
- Figs. 8a to c: a longitudinal cut through a first dispensing unit attachable to a first drug delivery device, a longitudinal cut through a second dispensing unit attachable to a second drug delivery device, and a longitudinal cut through a third dispensing unit attachable to a third drug delivery device;
- Fig. 9: a set of two drug delivery devices having different respective identifiers;
- Fig. 10: a further set of two drug delivery devices having different respective further kinds of identifiers;
- Fig. 11: a cartridge holder having a first kind of identifier;
- Fig. 12: a cartridge holder having a second kind of identifier;
- Fig. 13: a cartridge holder having a third kind of identifier;
- Fig. 14: a cartridge holder having a fourth kind of identifier;
- Fig. 15: a cartridge holder having a fifth kind of identifier;
- Fig. 16: a cartridge holder having a sixth kind of identifier;
- Fig. 17: a cartridge holder having a seventh kind of identifier;
- Fig. 18: a cartridge holder having a eighth kind of identifier; and
- Fig. 19: an electronic device attached at a drug delivery device.

In the present application, the term "distal part/end" refers to the part/end of the device, or the parts/ends of the components or members thereof, which in accordance with the use of the device, is located the furthest away from a delivery/injection site of a patient. Correspondingly, the term "proximal part/end" refers to the part/end of the device, or the parts/ends of the members thereof, which in accordance with the use of the device is located closest to the delivery/injection site of the patient.

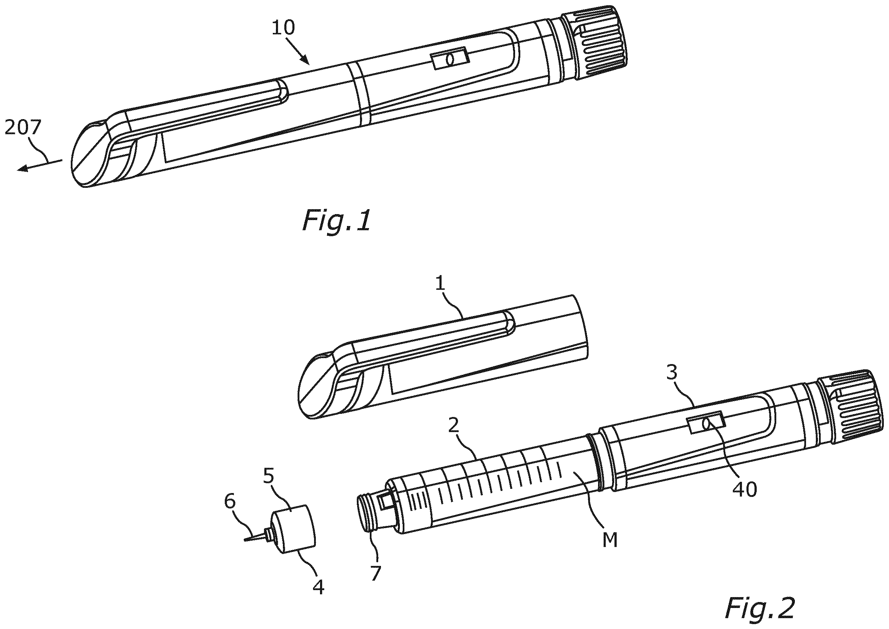

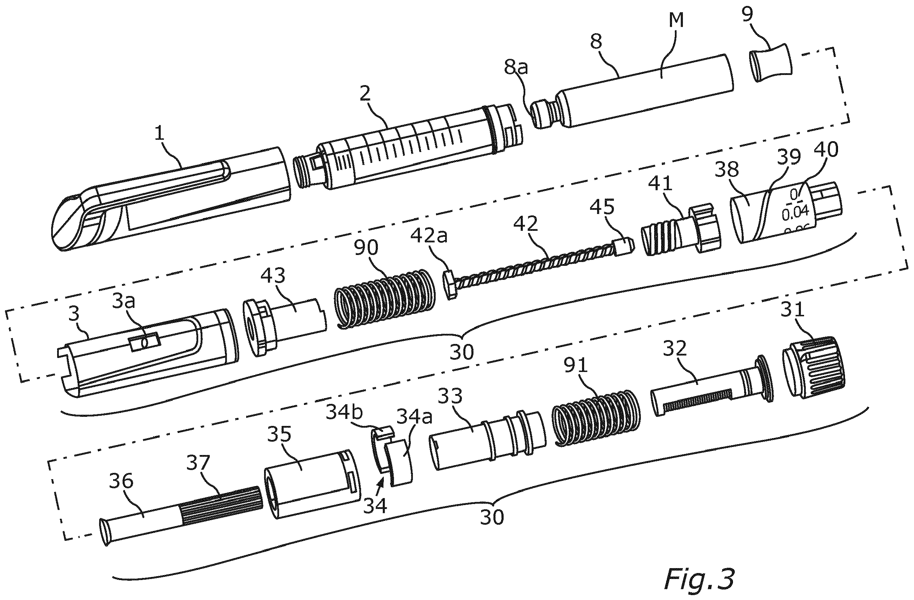

Fig.1 shows a perspective view of a disposable drug delivery device 10. The dose setting mechanism 30 (see Fig. 3) can be used in a number of variously designed drug delivery device 10. Fig. 1 shows the drug delivery device 10 in the zero dose state as indicated by indicia 40 showing a zero through the window 3a of housing 3.

Fig. 2 shows the device of Fig. 1 with cap 1 removed to expose the cartridge holder 2 and a proximal needle connector 7. A pen needle 4 is attached to the needle connector 7 through a snap fit, thread, Luer-Lok, or other secure attachment with a hub 5 such that a double ended needle cannula 6 can achieve a fluid communication with medicament contained M in cartridge 8. The cartridge 8 is sealed at the proximal end by septum 8a and with a sliding piston or plunger 9 at the opposite distal end.

The dose setting mechanism 30 has only a single component, namely dose selector 35, that is primarily responsible for determining a finite set of predetermined fixed doses within a maximum allowable dose range. Moreover, this finite set of predetermined fixed doses can contain fractional doses, meaning that each fixed dose does not have to be an equal multiple of the other fixed doses. For example, one fixed dose setting can equal an equal multiple of a lower fixed dose plus a fractional amount of that equal multiple.

The disposable drug delivery device 10 comprises an outer housing 3 that is rigidly connected to a piston guide 43 at its proximal end facing the injection site. The piston guide 43 guides a piston rod 42 that protrudes from the proximal end of the piston guide 43 and expels the medicament M from a cartridge 8 that is mounted to the proximal end of the piston guide 43 and the outer housing 3 via a cartridge container 2.

In this connection it should be noted that the medicament M can be present as a liquid or pasty substance M.

The piston rod 42 is rotationally fixed with respect to the piston guide 43 and the outer housing 3. The piston rod 42 has an outer thread that engages with an inner thread of a nut 36 that is disposed around the piston rod 42. The nut 36 is connected to a clutch 32 via a splined connection 37, whereby the nut 36 is rotationally fixed and axially movable with respect to the clutch 32.

At its proximal end the piston rod 42 comprises a foot 42a that engages the piston 9 of the cartridge 8. At its distal end the piston rod 42 comprises and abutment 45 that prevents the piston rod 42 from disengaging the nut 36 once the medicament M has been completely dispensed from the cartridge 8.

The clutch 32 is rigidly connected to a dose setting knob 31. The dose setting knob 31 is mounted to the housing 3 via a dose selector 35 that is axially movable and rotationally fixed with respect to the housing 3. Thereby, the knob 31 is axially fixed and rotationally movable with respect to the dose selector 35.

The drug delivery device 10 further comprises a dose sleeve 38 that has an outer thread, by which it is threadedly connected to the housing 3. Within the dose sleeve 38, the drug delivery device 10 comprises a driver 41, which is connected to the dose sleeve 38 by a splined connection so that the driver 41 is rotationally fixed and axially movable with respect to the dose sleeve 38. The driver 41 is furthermore threadedly connected to the piston guide 43. The driver surrounds the nut 36 and abuts, upon movement in the proximal direction, on a ridge on the proximal end of the nut 36. Therefore, proximal movement of the driver 41 also forces the nut 36 into the proximal direction.

A snap element 33 is axially and rotationally fixed to the dose sleeve 38, whereby the snap element 33 extends from the distal side of the dose sleeve 38. The snap element 33 surrounds the clutch 32 and the clutch 32, together with the knob 31 and the dose selector 35, is axially movable with respect to the snap element 33. Thereby, the knob 31 and the clutch 32 are biased into the distal direction by a compression spring 91 that acts between the snap element 33 and the clutch 32. Axial movement of the clutch 32 and the nut 31 is allowed until the knob 31 abuts the snap element 33 via the dose selector 35.

During dose setting, the clutch 32 and the nut 31 are in their distal position with respect to the snap element 33. In this position, the knob 31 is rotationally coupled to the snap element 33 via a teethed connection that comprises outer teeth at the distal end of the snap element 33 and inner teeth on the inner surface of the knob 31. When rotating the knob 31 during dose setting, the dose sleeve 38 is also rotated via the teethed connection between the knob 31 and the snap element 33 and screwed out of the housing 3. This forces the dose selector 35 and the knob 31 to also move in the distal direction. Rotation of the dose sleeve 38 also forces a corresponding rotation of the driver 41, which is therefore also screwed out of the piston guide 43.

Since the nut 36 is rotationally fixed with respect to the clutch 32, rotation of the knob 31 also causes rotation of the nut 36 during dose setting. Thereby, the nut 36 is screwed along the piston rod 42 and also moves into the distal direction. The pitches of the threads of the piston rod 42 and the driver 41 are adapted so that the nut 36 and the driver 41 essentially move the same axial distance upon rotation. Thereby, the nominal pitch of the connection between the driver 41 and the piston guide 43 is slightly higher than the nominal pitch of the thread between the piston rod 42 and the nut 36 to prevent mutual blocking of the nut 36 and driver 41 irrespective of manufacturing tolerances.

The threaded connection between the dose sleeve 38 and the housing 3 has a higher pitch than the connections between the piston rod and the nut 36 and the driver 41 and the piston guide 43, so that the dose sleeve 38 moves a larger distance than the driver 41 and the nut 36 upon rotation.

In this connection it should be noted that the dose sleeve 38 has an outer thread 39 that mates with a projection (not shown) present on an inner surface of the housing 3 during dose setting and dose delivery.

During dose setting, the snap element 33 rotates with respect to the dose selector 35. On its outer surface, the snap element 33 has a flexible arm with a radial protrusion, which engages with dose stops on the inner surface of the dose selector 35. The circumferential positions of the individual dose stops thereby define individual dose setting positions. To prevent the dialing of intermediate doses in between the individual dose stops, a torsion spring 90 is provided between the piston guide 43 and the driver 41.

This torsion spring 90 is loaded when increasing the set dose and causes the dose sleeve 38 to rotate back to the last set dose in cases where the dose setting knob 31 is released while the protrusion on the snap element 33 is positioned in between two dose stops.

To inject a set dose, the knob 31, the clutch and the dose selector 35 are moved into their proximal position with respect to the snap element 33. This releases the teethed connection between the snap element 33 and the knob 31 and engages a further teethed connection between the knob 31 and a connector 34 that surrounds the snap element 33.

The connector 34 is made of two parts 34a, 34b. In this connection it should be noted that the connector 34 may be of single piece design or of multi-piece design.

This connector 34 is rotationally movable and axially fixed with respect to the snap element 33. It is furthermore connected to the dose selector 35 via radially protruding ridges so that it is axially movable and rotationally fixed with respect to the dose selector 35. Engaging the teethed connection between the knob 31 and the connector 34 therefore prevents rotation of the knob 31 and clutch 32 with respect to the housing 3. This also rotationally fixes the nut 36 to the housing 3.

When further pushing the knob 31 into the proximal direction, the dose selector 35 abuts against the dose sleeve 38 and forces the dose sleeve 42 to move into the proximal direction. Due to the threaded connection between the dose sleeve 38 and the housing 3, the dose sleeve 38 rotates when moving into the proximal direction. This rotation is transferred to the driver 41, which is screwed into the proximal direction into the piston guide 43 and there-fore also moves axially in the proximal direction. The driver 41 thereby abuts and advances the nut 36, which is now rotationally fixed to the housing 3 and the piston rod 42 via the clutch 32, the knob 31, the connector 34 and the dose selector 35. Therefore, both the piston rod 42 and the nut 36 are rotationally fixed with respect to each other and axial advancement of the nut 36 causes a corresponding axial advancement of the piston rod 42, thus expelling the set dose.

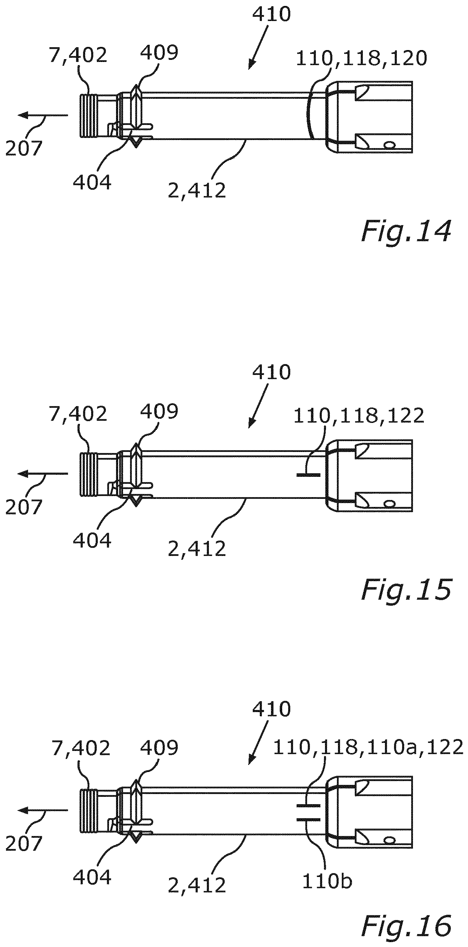

Further details on the drug delivery device 10, the dose setting mechanism 30 and in particular the specific design of the snap element 33 are disclosed in WO2019/011394A1 and in WO2020/015980 (see especially the disclosure of Fig. 15) whose contents are hereby incorporated by reference.

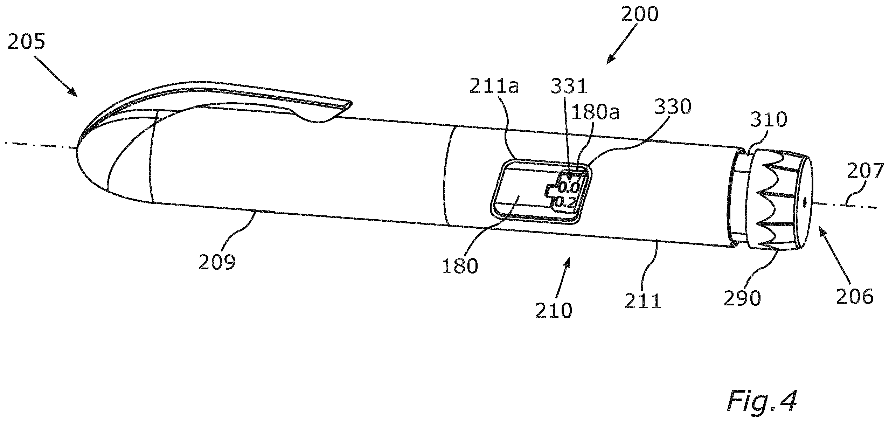

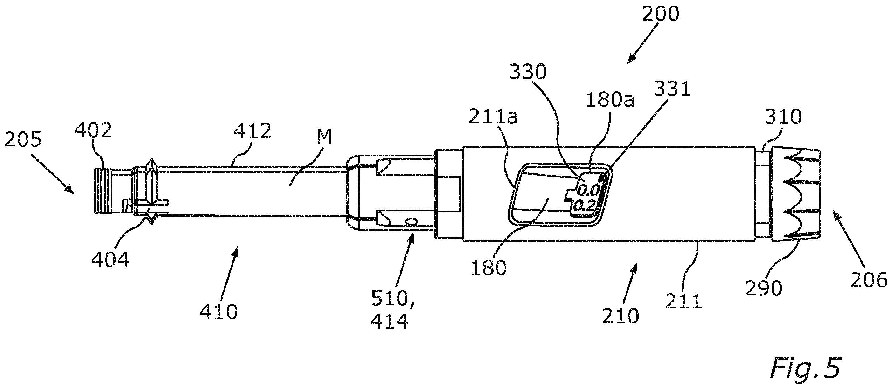

Fig. 4 shows a reusable drug delivery device 200 that comprises connection means for attaching a dispensing unit 410 (see e.g. Fig. 5). The drug delivery device 200 has a generally tubular housing 210, which is elongated along a longitudinal axis 207. A generally tubular cap 209 is attached to a proximal end 205 of the housing 210. At a distal end 206 of the housing 210, which distal end 206 is located opposite to the proximal end 205 along the longitudinal axis 207, the drug delivery device 200 comprises a dose setting member 290.

The dose setting member 290 is rotatable around the longitudinal axis 207 and is configured to be gripped and rotated by a user of the device 200 to set a dose to be delivered by the device 200. In the embodiment shown in Fig. 4, the dose setting member 290 is configured as a knob that terminates the drug delivery device 200 at its distal end 206.

The dose setting member 290 is connected to the housing 210 via a dose selector member 310 that is rotationally locked and axially movable relative to the housing 210 both during dose setting and during dose delivery. When increasing a set dose by turning the dose setting member 290, the dose selector member 310 moves distally out of the housing 210, thereby also moving the dose setting member 290 in the distal direction.

The housing 210 comprises an outer housing 211, which, in the present embodiment, is made from metal, and an inner housing 180. The inner housing 180 is located within the outer housing 211. In the present embodiment, it is made from a plastic material. The outer housing 211 comprises a window 211a through which a part of the inner housing 180 and a window 180a within the inner housing 118 is visible to a user of the device 200. Through the windows 211a and 180a, a dose indication member 330, which is located inside the generally tubular inner housing 180, is visible to the user.

The dose indication member 330 is also configured as a generally tubular member and carries on its outer cylindrical surface a dose scale comprising several optical markers 331 that correspond to the respective set dose. When setting a dose, the dose indication member 330 rotates within the inner housing 180, which changes the location of the scale and thus also the optical markers 331 visible through the windows 211a and 180a.

Fig. 5 shows the drug delivery device 200 with the cap 209 removed. A dispensing unit 410 that comprises the medicament to be delivered by the device 200 is removably attached to the proximal end 205 of the housing.

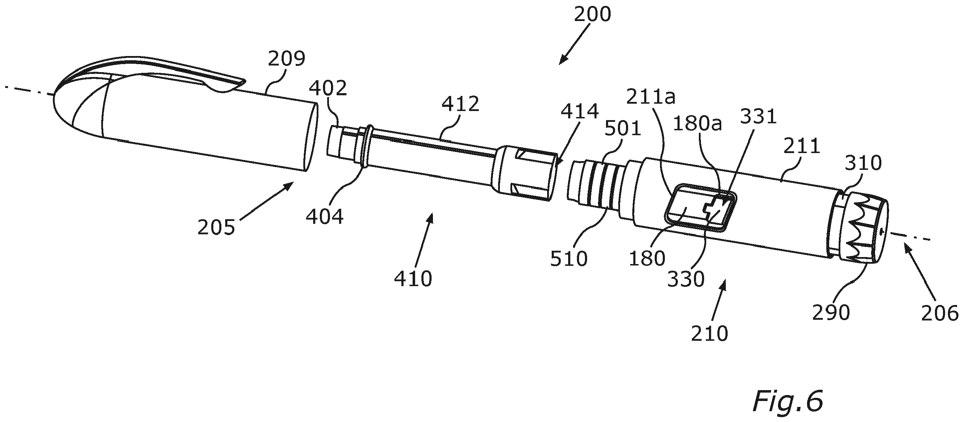

Fig. 6 shows the cap 209 and the dispensing unit 410 removed from the drug delivery device 200. With the cap 209 and the dispensing unit 410 attached to the housing 210 of the device 200, the dispensing unit 410 is fully received within the cap 209.

The dispensing unit 410 comprises a cartridge holder 412, which, in the current embodiment, is made from a plastic material. The cartridge holder 412 attaches to the outer housing 211 of the drug delivery device 200 via a connection, which comprises first connection means 510 located at the proximal end of the housing 210 and corresponding first connection means 414 located at the distal end of the dispensing unit 410. The first connection means 510 of the housing 210 are formed as integral part of the outer housing 211 and the first connection means 414 of the dispensing unit 410 are formed as integral part of the cartridge holder 412.

At its proximal end, the cartridge holder 412 of the dispensing unit 410 comprises a needle connector 402 that is configured to receive a hollow needle or cannula 6 through which the drug is delivered by the drug delivery device 200. In the present embodiment, the needle connector 402 is configured as a threaded connector.

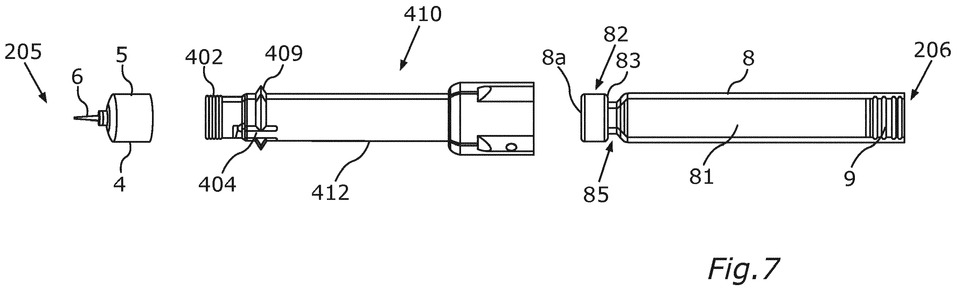

Fig. 7 shows the cartridge holder 412 of the dispensing unit 410 and a cartridge 8 that may be inserted into the cartridge holder 412, as well as a pen needle 4 attachable to the needle connector 402.

The cartridge 8 has a generally cylindrical body, which, in the present embodiment, is made from glass, and which surrounds a drug compartment 81 that contains a liquid drug M to be delivered by the drug delivery device 200. The drug compartment 81 is sealed at its distal end by an elastic plunger 9, which is movable along the longitudinal axis within the body of the cartridge 8. At its proximal end, the cartridge 8 comprises an annular rim 82, which is separated from the body by an annular detent 85 located distally from the annular rim 82. At a proximal front surface of the cartridge 8, which is orientated perpendicular to the longitudinal axis 207, the cartridge 8 comprises a sealing means or septum 8a, which seals the drug compartment 81 in the proximal direction.

When being fully inserted into the cartridge holder 412, the sealing means 8a is located at the proximal end of the cartridge holder 412 and accessible through an opening of the cartridge holder 412. The cartridge 8 is non-releasably held in its inserted position by a snap hook 404. The snap hook 404 is configured as a flexible member. In the present embodiment, the snap hook 404 is formed by a cut-out portion of the cartridge holder 412. Upon insertion of the cartridge 8 into the cartridge holder 412, the snap hook 404 snaps over the annular rim 82 of the cartridge 8. A radially inwardly protruding finger of the snap hook 404 is then located within the annular detent 85 of the cartridge 8 and prevents distal movement of the cartridge 8 by abutting against a distal surface 83 of the annular rim 82.

This non-releasable connection between the cartridge 8 and the cartridge holder 412 prevents a removal of the cartridge 8 from the cartridge holder 412 during intended use of the dispensing unit 410. For example, it prevents removal of the cartridge container 8 unless the snap hook 404 is intentionally and forcefully brought out of engagement with the annular rim 82. The snap hook 404 is thereby configured in a way that such disengagement is only possible using tools or excessive forces that are higher than the forces acting on the snap hook 404 during normal and/or intended use of the dispensing unit 410, for example during mounting of the dispensing unit 410 to the housing 210, during attachment of the pen needle 4 to the cartridge container 412 or during handling of the dispensing unit 410 with the cartridge 8 inserted into the cartridge holder 412. This handling may also comprise shock forces that may occur during transport and/or unintentional dropping of the dispensing unit and that do not exert forces that would destroy the dispensing unit 410 and/or the cartridge holder 412 and/or the cartridge 8. The non-releasable connection between the cartridge 8 and the cartridge holder 412 allows to provide and sell the dispensing unit 410 with an inserted cartridge 8 as a single, pre-mounted unit.

The hub 5 comprises at its distal end a hub connector that matches the needle connector 402 of the cartridge holder 412 With its distal end, the cannula 6 penetrates the sealing means 8a of the cartridge 8 and thus establishes a fluid connection between the drug compartment 81 and the proximal end of the cannula 6. The proximal end of the cannula 6 is configured to be inserted into a delivery site, such as a skin of the user of the device 200, thereby permitting injection of the drug into the delivery site.

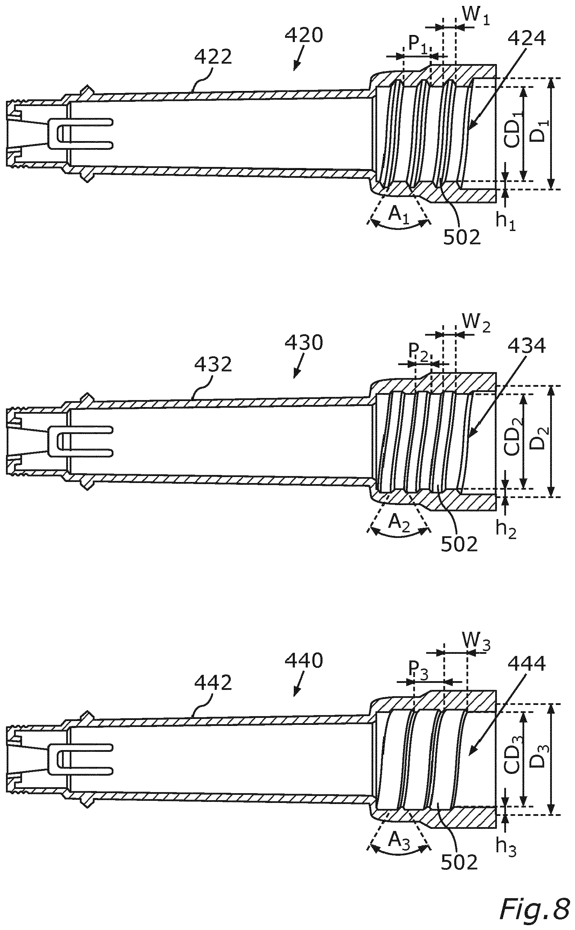

Fig. 8 shows a set of three dispensing units according to the present disclosure. Each drug delivery device is connected to its corresponding dispensing unit by a keyed connection that prevents the respective drug delivery device from connecting to the other dispensing units and which, vice versa, prevents the corresponding dispensing unit from connecting to the other drug delivery devices.

Fig. 8 shows a longitudinal cut through a first dispensing unit 420 attachable to the housing 211 of a first drug delivery device 200 via first connection means 424 of a first cartridge holder 422, a longitudinal cut through a second dispensing unit 430 attachable to a second housing 211 of a second drug delivery device 200 via second connection means 434 of a second cartridge holder 432 of the second dispensing unit 430 and a longitudinal cut through a third dispensing unit 440 attachable to a third housing 211 of a third drug delivery device 200 via third connection means 444 of a third cartridge holder 442 of the third dispensing unit 440.

The connection means 424, 434, 444 of the cartridge holders 422, 432, 442 and the corresponding connection means 510 of the drug delivery devices 200 form keyed connectors. Thereby, the connection means 424, 434, 444 are of the same type and the connection means 510 are also of the same type.

The individual connection means 424, 434, 444 of the cartridge holders 422, 432, 442 each form female parts of the connections and the individual connection means 510 of the drug delivery devices 200 form corresponding male parts. All connection means 424, 434, 444, 510 are configured as threads, whereby the connection means 424, 434, 444 of the cartridge holders 422, 422, 432 form inner threads and the connection means 510 of the drug delivery devices 200 form outer threads.

The geometries of the threads 424, 434, 444, 510 are defined by several thread dimensions. The thread dimensions comprise a core diameter or minor diameter that specifies the minimum inner diameter of the female part of the connections, an outer diameter or major diameter that specifies the maximum inner diameter of the female part of the connections, a pitch that specifies a distance between adjacent ridges 501 or valleys 502 of the threads, a width of the ridges 501 (see Fig. 6) provided on the male part of the threads, which corresponds to a width of the valleys 502 provided on the female part of the threads and an opening angle between sidewalls of adjacent ridges 501 of the male parts. A height of the ridges 501 of the male parts and a corresponding height of the valleys 502 of the female parts is given by the difference between the outer diameter and the core diameter.

Unless stated otherwise, the term "ridges" used in the present disclosure always refers to the ridges 501 of the male thread of a given threaded connection, irrespective of whether the part being described actually comprises a male thread or a female thread.

Keying is achieved by at least one of the thread dimensions, such as at least one of the core diameter, the outer diameter, the pitch, the width of the ridges 501 and the opening angle, being mutually different among the individual pairs of corresponding connection means 424, 434, 444, 510 of the cartridge holders 422, 432, 442 and drug delivery devices 220.

With the embodiments shown in Fig. 8, the only thread dimensions that differ among the individual dispensing units 420, 430, 440 and therefore also among the individual drug delivery devices 200 are the width and the height of the individual ridges 501 of the male parts and the corresponding widths and heights of the valleys 502 of the female parts. Thereby, the ridges 501 of the first connection means 511 have a first width w1, the ridges 501 of the second connection means 520 have a second width w2, and the ridges 501 of the third connection means 530 have a third width w3. The first width w1 is smaller than the second width w2, and the second width w2 is smaller than the third width w3. Exemplarily, the second width w2 is twice the first width w1 and the third width w3 is three times the first width w1.

Furthermore, the ridges 501 of the first connection means 510 have a first height, the ridges 501 of the second connection means 510 have a second height, and the ridges 501 of the third connection means 510 have a third height. The first height is larger than the second height, and the second height is larger than the third height. Thereby, the first height is twice the second height and the first height is three times the third height.

The aforementioned differences in the heights, combined with the aforementioned differences in the widths w1, w2, w3 reliably prevent mounting of the individual dispensing units 420, 430, 440 to other than their corresponding drug delivery device 200 with the matching connection means 511.

With the embodiments shown in Fig. 8, a first pitch P1 of the first connection means 424, a second pitch P2 of the second connection means 434 and a third pitch P3 of the third connection means 444 are the same. Furthermore, a first angle A1 of the first connection means 424, a second angle A2 of the second connection means 434 and a third angle A3 of the third connection means 444 are also the same.

Further details on the drug delivery device 200, the dose setting mechanism thereof as well as the keying features are disclosed in EP21167293.6 whose contents are hereby incorporated by reference.

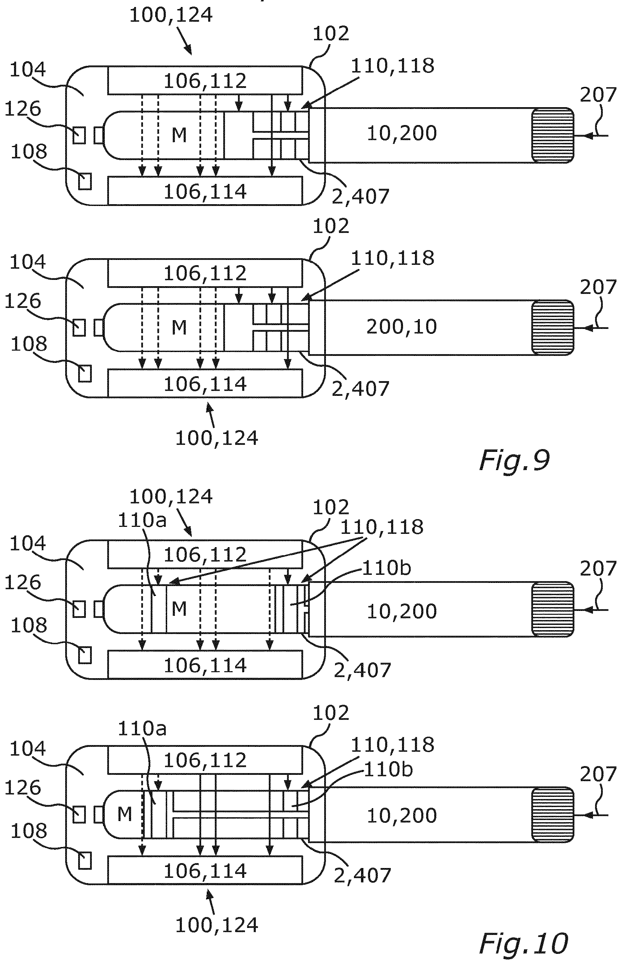

Fig. 9 shows a set of two drug delivery devices 10, 200 having different respective identifiers 110 arranged at different axial positions along the longitudinal axis 207 at a surface 116 of a part of the housing of the drug delivery device 10, 200.

In the present example the identifiers are arranged at different axial positions along the longitudinal axis 207 at the cartridge holder 2, 407.

In this connection it should be noted that the cartridge holder 2, 412 could be configured to receive a cartridge 8 that comprises the drug compartment 8, 81 or could be integrally formed with the cartridge 8.

In this connection it should be noted that the surface 116 bearing the identifier is preferably an outer surface visible to a user and other than a surface of the cartridge holder 2, 407 can also be a surface of the cartridge 8, a surface of a dispensing unit 410, and a surface of the housing 3, 211.

The cartridge holder 2, 407 is inserted in to a cavity 104 of an electronic device 100. The electronic device 100 further comprises a housing 102 having the cavity 104, a sensor assembly 106, and a processing unit 108. The sensor assembly 106 and the processing unit 108 are arranged inside the housing 102. The sensor assembly 106 comprises an emitter unit 112 and a detector unit 114.

In this connection it should be noted that the emitter unit 112 is selected from the group of members comprising one single emitter, a unit comprising several emitters and several emitters working as a unit. By way of example the emitter unit 112 can be formed by one or more light emitting diodes (LEDs) arranged at a side of the cartridge holder 2, 407 remote from the detector unit 114.

In this connection it should be noted that the LEDs of the emitter unit 112 could be infrared emitters, with the sensor assembly 106 then comprising an infrared detector unit 114.

By way of example, the emitter unit 112 could be configured as an emitter array comprising several longitudinally and optionally horizontally spaced-apart emitter elements each emitting electromagnetic radiation of a specific wavelength, with the wavelengths being the same as or different from the wavelengths emitted by the other emitters in the array.

In this connection it should further be noted that the detector unit 114 is selected from the group of members one single detector, a detector unit comprising several individual detectors and several detectors working as a common detector unit.

By way of example, the detector unit 114 could be configured as a position resolving detector, such as a detector array comprising several longitudinally and optionally horizontally spaced-apart detector elements.

In this connection it should be noted that the detector unit 114 may comprise between 8 and 256 detector elements, in particular wherein the detector unit 114 may comprise between 16 and 64 detector elements.

In order to determine an amount of medication M stored in the cartridge 8, the electromagnetic radiation emitted by the emitter unit 112 is emitted into the cavity 104. The emitted electromagnetic radiation is incident on the moveable plunger 9 and reflects and scatters off the moveable plunger 9 sealing the drug compartment 8, 81.

The reflected and scattered light causes the light distribution detected by the detector unit 114 to differ for different axial positions of the moveable plunger 9. The detector unit 114 measured these different respective light intensities over the length of the detector unit 114.

The processing unit 108 is then configured to distinguish between the different measured light intensities and to output a detected amount of medication M. Hence, the volume of the liquid M inside the drug compartment 8, 81 is determined by determining a longitudinal position of the plunger 9 within the drug compartment 8, 81.

It is preferable if the housing 102 of the electronic device 100 is configured as a removable cap 124 of the drug delivery device 10, 200, wherein the removable cap 124 is configured to surround a proximal delivery end of the drug delivery device 10, 200.

It is further preferred if the cap is configured to be attachable to the drug delivery device 10, 200 in a single rotational position only.

The emitting of the electromagnetic radiation into the cavity 104 and the receiving of the electromagnetic radiation from the cavity 104 can be performed while the drug compartment 8, 81 and the identifier 110 are received stationary inside the cavity 104.

Alternatively the emitting of the electromagnetic radiation into the cavity 104 and the receiving of the electromagnetic radiation from the cavity 104 is performed while the drug compartment 8, 81 and the identifier 110 are moved into and/or out of the cavity 104.

The identifier 110 is located at a distal end 206 of the drug compartment 8, 81 on the cartridge holder 2, 407 facing away from a delivery end of the drug delivery device 10, 200, i.e. facing away from the end of the drug delivery device 10, 200 at which the cannula 6 is attached.

The identifier 110 is located at a longitudinal position that radially overlaps with the drug compartment 8, 81 and hence the cartridge holder 2, 407. In this way the electronic device so to say also houses the identifier 110 when the part of the drug delivery device 10, 200 bearing the identifier 110 is inserted into the cavity.

The drug delivery devices 10, 200 that can be used with the electronic device 100 can be disposable drug delivery devices 10, such as the ones discussed in connection with Figs. 1 to 3 and reusable drug delivery devices 200 as discussed in connection with Figs. 4 to 8.

The drug delivery devices 10, 200 shown in Fig. 9 can be of the same kind of drug delivery device and/or be different from one another. The feature that primarily distinguishes between the two drug delivery devices 10, 200 is the identifier present on the cartridge holder 2, 407.

In this connection it should be noted that the electronic device 100 could also be used to detect the type of cartridge holder 2, 407 inserted into the cavity 104. This is particularly beneficial in connection with the reusable drug delivery device 200.

In this instance the following method can also be adapted to detect the kind of cartridge holder 2, 407 rather than the type of drug delivery device 10, 200. This is made possible, as the cartridge holder 2, 407 if this is configured to hold a cartridge 8 does not permit a simple removal of the cartridge 8. In this way the electronic device 100 can be configured to distinguish between different medicaments M stored in the respective cartridge holder 2, 407 rather than simply distinguishing between different drug delivery devices 10, 200.

Fig. 10 shows a further set of two drug delivery devices 10, 200 having different respective further kinds of identifiers 110.

The drug delivery devices 10, 200 shown in Fig. 10 can also be of the same kind of drug delivery device and/or be different from one another. The feature that primarily distinguishes between the two drug delivery devices 10, 200 is the identifier present on the cartridge holder 2, 407.

The identifier 110 shown in Fig. 10 comprises a first and second portion 110a, 110b, wherein the first and second portion 110a, 110b are spaced apart from each other along the longitudinal axis 207 of the drug delivery device 10, 200.

The first and second portions 110a, 110b can also differ in size and/or shape in order to permit further permutations of the kinds of drug delivery devices and/or medicaments M used in connection with the electronic device 100.

Rather than selecting different spacings between the identifiers 110, at least one of the first and second portions 110a, 110b of the identifiers 110 and preferably both of the first and second portions 110a, 110b could also differ in size and/or shape at given spacings. This means that the first portion 110a, could always be of identical size and shape at the identical axial position along the longitudinal axis 207. The second portion 110b then has a varying size and/or shape at a given position.

In this connection the spacings between first and second portions 110a, 110b can be constant with both the first and second portions 110a, 110b differing in size and/or shape.

Optionally a distance between the first and second portion is larger than a longitudinal extent of a movable plunger 9 sealing the drug compartment 8, 81.

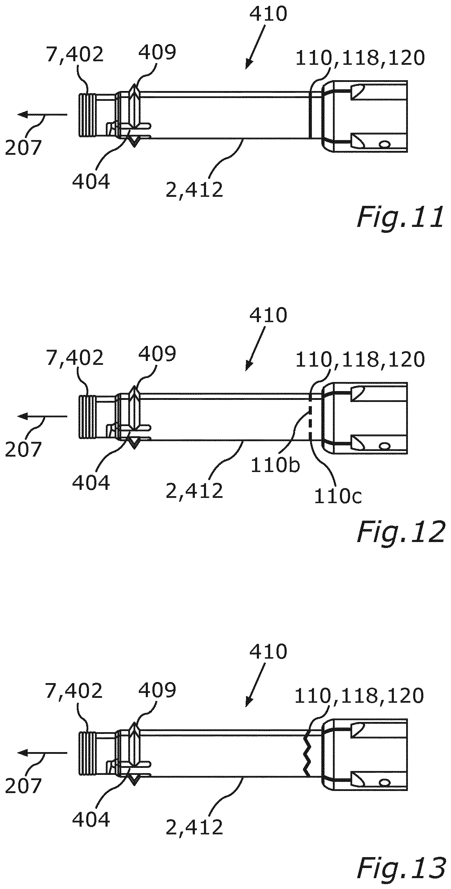

Fig. 11 shows a cartridge holder 2, 407 having a first kind of identifier 110. The identifier 110 comprises a circumferential marker 120 that surrounds the longitudinal axis 207 of the drug delivery device 10, 200.

Fig. 12 shows a cartridge holder 2, 407 having a second kind of identifier 110. In this instance, the identifier 110 is also a circumferential marker 120 that comprises a plurality of portions 110a, 110b, 110c. Each portion 110a, 110b, 110c has a circumferential extent. The individual portions 110a, 110b, 110c are spaced apart from one another along the circumference of the cartridge holder 2, 407 transverse to the longitudinal axis 207.

The spacing around the circumference can be constant or different between the different portions 110a, 110b, 110c.

It should also be noted that the length of the different portions 110a, 110a, 110c and/or their width can vary in order to provide cartridge holder 2, 407 respectively drug delivery device 10, 200 specific identifiers.

It should also be noted, that the axial positions of the portions 110a, 110b, 110c of the circumferential markers 120 can vary slightly within a given bandwidth when selecting such identifiers 110.

Fig. 13 shows a cartridge holder 2, 407 having a third kind of identifier 110. The circumferential marker 120 forming the identifier 110 is of zig-zag shape. Different identifiers 110 having zigzag shapes can have different axial and radial positions of the peaks and crests of the circumferential marker in relation to the longitudinal axis 207.

Also a thickness of the circumferential marker 120 can vary for different kinds of drug delivery devices 10, 200, respectively cartridge holders 2, 407.

Fig. 14 shows a cartridge holder 2, 407 having a fourth kind of identifier 110. The circumferential marker 120 is not formed by a straight line that extends perpendicular to the longitudinal axis 207 about the cartridge holder 2, 407 as is the case for the marker shown in Figs. 9 to 11, but is curved in relation to such a straight line and the longitudinal axis 207.

Different drug delivery devices 10, 200 can be provided with circumferential markers 120 arranged at different axial positions along the longitudinal axis 207, with the different axial positions being associated with specific types of drug delivery devices 10, 200 and/or medicaments M stored therein.

Additionally or alternatively different drug delivery devices 10, 200 can be provided with circumferential markers 120 that have different sizes, e.g. thicknesses (not shown) arranged at the same and optionally different axial positions along the longitudinal axis 207, with the different sizes and optionally the different axial positions being associated with specific types of drug delivery devices 10, 200 and/or medicaments M stored therein.

Additionally or alternatively different drug delivery devices 10, 200 can be provided with circumferential markers 120 that have different shapes arranged at the same and optionally different axial positions along the longitudinal axis 207 and/or optionally having different sizes, with the different shapes and optionally the sizes and/or different axial positions being associated with specific types of drug delivery devices 10, 200 and/or medicaments M stored therein.

Fig. 15 shows a cartridge holder having a fifth kind of identifier 110 formed as a longitudinal marker 122. The longitudinal marker 122 only covers a limited radial sector around a longitudinal axis of the drug delivery device 10, 200.

In order to differentiate between different longitudinal markers 122 or between longitudinal markers 122 and circumferential markers 120, the respective longitudinal markers 122, can have different lengths along the longitudinal axis 207, and/or different thicknesses in parallel to the longitudinal axis 207.

Fig. 16 shows a cartridge holder 2, 407 having a sixth kind of identifier 110. The longitudinal marker 122 is formed by first and second portions 110a, 110b that have a generally elongate extent in parallel to the longitudinal axis 207 and that are arranged in parallel to one another about the circumference of the cartridge holder 2, 407.

A length and/or thickness of the first and second portions 110a, 110b of the longitudinal markers 122 can vary in order to differentiate between different drug delivery devices 10, 200 respectively cartridge holders 2, 407.

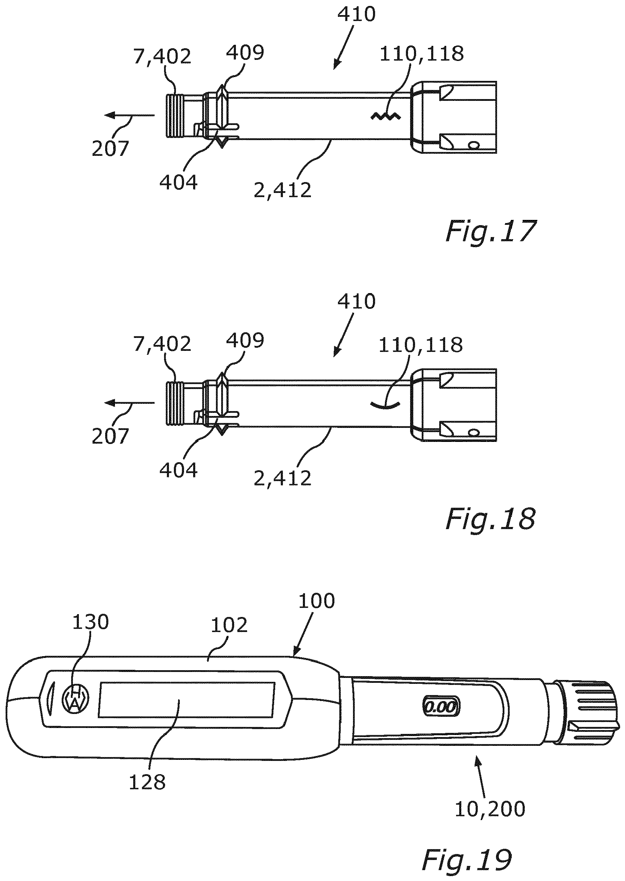

Fig. 17 shows a cartridge holder 2, 407 having a seventh kind of identifier 110. The longitudinal marker 122 forming the identifier 110 is of zig-zag shape. Different identifiers 110 having zigzag shapes can have different axial and radial positions of the peaks and crests of the longitudinal marker 122 in relation to the longitudinal axis 207.

Also a thickness of the longitudinal marker 122 can vary for different kinds of drug delivery devices 10, 200, respectively cartridge holders 2, 407.

Fig. 18 shows a cartridge holder 2, 407 having an eighth kind of identifier 110. The longitudinal marker 122 is not formed by a straight line that extends in parallel to the longitudinal axis 207 at the cartridge holder 2, 407 as is the case for the marker shown in Figs. 15 and 16, but is curved in relation to the longitudinal axis 207.

Different drug delivery devices 10, 200 can be provided with longitudinal markers 122 arranged at different circumferential positions in relation to the longitudinal axis 207, with the different circumferential positions being associated with specific types of drug delivery devices 10, 200 and/or medicaments M stored therein.

Additionally or alternatively different drug delivery devices 10, 200 can be provided with longitudinal markers 122 that have different sizes, e.g. thicknesses (not shown) arranged at the same and optionally different circumferential positions in parallel the longitudinal axis 207, with the different sizes and optionally the different circumferential positions being associated with specific types of drug delivery devices 10, 200 and/or medicaments M stored therein.

Additionally or alternatively different drug delivery devices 10, 200 can be provided with longitudinal markers 122 that have different shapes arranged at the same and optionally different circumferential positions in parallel to the longitudinal axis 207 and/or optionally having different sizes, with the different shapes and optionally the sizes and/or different circumferential positions being associated with specific types of drug delivery devices 10, 200 and/or medicaments M stored therein.

The electronic device 100 is preferably configured to be able to differentiate between the different kinds of identifiers 110, regardless of whether these are configured as longitudinal markers 122 or circumferential markers 120.

In this connection it should be noted that the identifier is a coding feature for a specific a drug delivery device 10, 200, respectively cartridge holder 2, 407. In use of the electronic device 100 the electronic device 100 is configured to decode the coding feature by decoding its axial and/or circumferential position and/or its shape and size in order to determine the type of a drug delivery device 10, 200 respectively the cartridge holder 2, 407 inserted into the cavity 104 of the electronic device 100.

Generally speaking the coding feature may comprise a member selected from the group of members consisting of one or more straight lines of different size and/or colour, one or more curved lines of different size and/or colour, one or more zigzag lines of different size and/or colour, one or more dashed lines of different size and/or colour, fluorescent markings, a colored shape, a transparent code that filters only part of the spectrum of the electromagnet radiation; and combinations of the foregoing.

The coding feature may comprise one or more dimensions of the identifier 110 and/or specifics, such as shape, of the identifier 110, with the one or more dimensions of the identifier 110 and/or specifics, such as shape, of the identifier 110 being detectable by the sensor assembly 106, i.e. the detection unit 114 that is also configured to detect a position of a plunger 9 present within the drug compartment 8, 81.

In this connection it should be noted that a width of the identifier may be selected in the range of 0.1 mm to 20 mm, preferably in the range of 0.5 mm to 15 mm.

In this connection it should also be noted that a length of the identifier may be selected in the range of 4 mm to 40 mm, preferably in the range of 5 mm to 20 mm.

It should further be noted that a length of the circumferential marker 120 is typically selected in the range of 60 to 100 of an outer diameter of the component at which the circumferential marker 120 is attached, i.e. a range in which the circumferential marker 120 at least partly surrounds the component at which it is applied to completely surrounding the component at which the circumferential marker 120 is applied.

The identifier 110 may be provided on a housing part of the housing 3, 211 surrounding the drug compartment 8, 81, and/or at the cartridge holder 2, 407 and the housing part and/or the cartridge holder 2, 407 may then be transparent to the electromagnetic radiation.

It should also be noted that the identifier 110 may comprise a pattern 118 with a modulated refractive index at a wavelength of the electromagnetic radiation. This enhances a detection of the light scattered and/or reflected at the identifier 110 and thereby improves the reliability of detection.

The pattern 118 may provide a modulated attenuation of the electromagnetic radiation, wherein the detector signal may then represent a shadow cast by the identifier 110 on the detector unit 114.

Fig. 19 shows the electronic device 100 configured as the cap 124 attached to the drug delivery device 10, 200, i.e. the cartridge holder 2, 412 of the drug delivery device 10, 200 is inserted into the cavity 104 of the electronic device 100.

The electronic device has a front side 132 having a display 128 and a button 130, such as a touch button, arranged at the front side. In use of the electronic device 100 this can be switched on and off by a user of the electronic device 100 via the button 130.

On switching on the electronic device 100, the display 128 is configured to display a remaining dose within a drug delivery device 10, 200 and/or the type of drug delivery device 10, 200 inserted into the cavity 104 of the electronic device.

The housing of the electronic device 100 houses not only the sensor assembly 106 comprising the emitter unit 112 and the detector unit 114, but also the processing unit 116 arranged on a non-shown mainboard, the memory 126, a battery (not shown), an optionally provided interface, such as a USB Port and/or a Bluetooth interface (not shown) or the like, such as Wifi (also not shown) and flex cables (not shown) connecting the different components one to another electrically.

If provided the USB Port is typically provided at a proximal side 134 of the electronic device. The proximal side 134 is arranged transverse to the front side 132.

In this connection it should be noted that the electronic device can be configured to receive the cartridge holder 2, 412 with or without a needle connector 7, 402 having the corresponding cannula 6 attached.

A set of a drug delivery device 10, 200, such as the one shown in Fig. 19 comprises the electronic device 100 configured to carry out a method as explained in the foregoing.

The electronic device 100 can also be configured to interact with a smart device (not shown), such as a smart watch, phone and/or tablet, e.g. via WLAN, Bluetooth, or the like, with the smart device similarly being configured to carry out a method as described herein.

The smart device can then configured to store measured amounts of medicament M associated with a kind of drug delivery device 10, 200 and to display said measured amounts of the medicament M in dependence on time and drug delivery device 10, 200. In this way a user of the drug delivery device 10, 200 or a medical practitioner overseeing the use of the drug delivery device 10, 200 can track a user's behavior over time and adapt dosage regimes of the user.

In use of the electronic device 100 the drug compartment 8, 81, i.e. the cartridge holder 2, 412 having the identifier 110 is received inside the cavity 104 of the housing 102 of the electronic device 100. During the insertion and/or thereafter electromagnetic radiation is emitted from the emitter unit 112 into the cavity 104. At least part of the electromagnetic radiation from the cavity 104 is incident at and received with the detector unit 114. A detector signal is then generated that represents the electromagnetic radiation received by the detector unit 114. From the detector signal a volume of the liquid M present inside the drug compartment 8, 81 is determined using the processing unit 108. At the same time or shortly before or after the detection of the volume of the liquid/medicament inside the drug compartment is measured a presence of the identifier 110 is also detected from the detector signal using the processing unit 108; and based on the information coded into the identifier, be it through the shape and/or size and/or position of the identifier, the electronic device 100 distinguishes, using the processing unit 108, the drug delivery device 10, 200 based on the identifier 110 from a further drug delivery device 10, 200 having a further identifier 110 that is different from the identifier 110. Generally speaking the identifier 110 is provided on, for example printed on or glued to, a surface 116, preferably an outer surface 116, of the drug delivery device 10, 200.

The processing unit 108 determines the volume of the liquid M from a first signal component of the detector signal and detects the identifier 110 from a second signal component of the detector signal different from the first signal component.

For example, the signal components amount to signals generated by spatially separated sensor elements of the detection unit 114. This is possible as the light is scattered at the identifier 110 into different spatial directions in dependence in the size and/or shape and/or position of the identifier 110. By using e.g. 6 to 20 such spatially separated sensor elements of the detection unit 114, the different scattering intensities of the emitted electromagnetic radiation can be used to identify the identifier 110 and through a comparison, e.g. with stored calibration data permits a reliable detection of the kind of identifier 110 and hence of the kind of drug delivery device 10, 200, the cartridge holder 2, 412 and/or medicament M associated with either of these.

The electronic device 100 may comprise the memory 126 that stores at least two different sets of configuration data, each set of configuration data being associated with a different type of drug delivery device 10, 200 and/or liquid M, and/or cartridge holder 2, 412. From the set of configuration a selected set is selected using the processing unit 108 based on the identifier 110 detected by the processing unit 108.

The configuration data may comprise calibration data, wherein the volume of the liquid M inside the drug compartment 8, 81 may be determined by the processing unit 108 using the calibration data of the selected set.

In order to avoid an attempt at detecting the wrong amount of medication M present in the cartridge 8, the electronic device 100 may be configured to compare a type of the identified drug delivery device 10, 200 with a predetermined type, and to generate and/or to output a warning if the type of the identified drug delivery device 10, 200 deviates from the predetermined type.

Similarly the electronic device 100 may be configured to receive a foreign object having no identifier 110 inside the cavity 104 of the housing 102 of the electronic device 100; and to generate and/or to output a warning by the processing unit 108 upon a failed detection of an identifier 110 after receiving the foreign object.

The cap 124 and its sensors (detection unit 114) are designed to cover the whole cartridge container 2, 412. In the case that the drug delivery device is not filled completely per default, additional markings can be printed on the back part of the cartridge container.

The presence and position of these markings can be assigned to the dedicated type of drug delivery device 10, 200 and can be are used to identify the inserted drug delivery device 10, 200. These additional markings can be positioned in dependence on the kind of drug delivery device. The identifiers 110 should be as large as possible for a reliable detection, but small enough to not conflict with the measurement of the liquid level.

In the that identifiers 110 having several portions 110a, 110b, 110c are provided these additional portions are provided as a fail-safe mechanism. The additional markings, i.e. portions are repeated at least 2 times at longitudinal spacings on the cartridge container. At least one bundle of markings, i.e. portions 110a, 110b, 110c should not be affected by the plunger 9.

In general the cap 124 is used in that the cap 124 is attached to the drug delivery device 10, 200. The user then removes the cap 124 and sets a dose of the drug delivery device 10, 200. Thereafter the user reattaches the cap 124 at the drug delivery device 10, 200. The cap 124 detects the identifier 110, the cap 124 then returns in addition to the existing data (timestamp, dose and temperature) a value for the type of drug delivery device 10, 200.

This permits the algorithm running on the processing unit 108 to switch between pre-programmed drug delivery device configurations or calibration sets.

This enables the overall system to trigger user guidance and warnings (e.g. the wrong drug delivery device is used).
Alternatively, it would be possible add a dedicated RGB colour sensor into the additional device (e.g. connected cap) which checks coloured markings at the cartridge container 2, 412, on the drug delivery device or already existing coloured structures of the drug delivery device 10, 200.

As shown above the presented solution allows the simple detection of a low number of different types of drug delivery devices 10, 200 (least 3) and foreign objects which do not hold any of the identifiers.

The presented variants see e.g. Figs. 9 to 16A and B can be implemented direct in the Firmware (Software) of existing Connected Caps without any changes of the hardware. There is only the need to print the additional markings, identifiers 110 on the drug delivery devices 10, 200, such as on the cartridge holders 2, 412.

### List of reference numerals:

- 1: cap
- 2: cartridge holder
- 3: housing
- 3a: window
- 4: pen needle
- 5: hub
- 6: cannula
- 7: needle connector
- 8: cartridge
- 8a: septum
- 9: piston
- 10: disposable drug delivery device
- 30: dose setting mechanism
- 31: knob
- 32: clutch
- 33: snap element
- 34: connector
- 34a, 34b: part of 34, part of 34
- 35: dose selector
- 36: nut
- 37: splinned connection
- 38: dose sleeve
- 39: outer thread on 38
- 40: indicia
- 41: driver
- 42: piston rod
- 42a: foot
- 43: piston guide
- 45: abutment
- 81: drug compartment
- 82: annular rim
- 83: distal surface
- 85: annular detent
- 90: torsion spring
- 91: compression spring

- 100: electronic device
- 102: housing
- 104: cavity
- 106: sensor assembly
- 108: processing unit
- 110: identifier
- 110a: first identifier
- 110b: second identifier
- 110c: third identifier
- 112: emitter unit
- 114: detector unit
- 116: surface of 10, 200
- 118: pattern
- 120: circumferential marker
- 122: longitudinal marker
- 124: cap
- 126: memory
- 128: display
- 130: button
- 132: front side
- 134: proximal side

- 180: inner housing
- 180a: window
- 200: drug delivery device
- 205: proximal end
- 206: distal end
- 207: longitudinal axis
- 209: cap
- 210: housing
- 211: outer housing
- 211a: window
- 290: dose setting member
- 310: dose selector member
- 330: dose indication member
- 331: optical marker
- 402: needle connector
- 404: snap hook
- 410: dispensing unit
- 412: cartridge holder
- 414: connection means
- 420: first dispensing unit
- 422: first cartridge holder
- 424: first connection means
- 430: second dispensing unit
- 432: second cartridge holder
- 434: second connection means
- 440: third dispensing unit
- 442: third cartridge holder
- 444: third connection means
- 501: ridge
- 502: valleys
- P₁: first pitch
- W₁: first width
- CD₁: first core diameter
- D₁: first outer diameter
- A₁: first angle
- P₂: second pitch
- W₂: second width
- CD₂: second core diameter
- D₂: second outer diameter
- A₂: second angle
- P₃: third pitch
- W₃: third width
- CD₃: third core diameter
- D₃: third outer diameter
- A₃: third angle
- M: Medicament

### Enumerated embodiments:

1. Method for identifying a drug delivery device (10, 200), such as a pen-injector, using an electronic device (100),
   wherein the electronic device (100) comprises a housing (102) having a cavity (104), a sensor assembly (106), and a processing unit (108),
   wherein the sensor assembly (106) and the processing unit (108) are arranged inside the housing (102),
   wherein the drug delivery device (10, 200) comprises a drug compartment (8, 81) for holding a liquid (M) to be delivered by the drug delivery device (10, 200) and an identifier (110),
   wherein the sensor assembly (106) comprises an emitter unit (112) and a detector unit (114),
   wherein the method comprises:
   - receiving the drug compartment (8, 81) and the identifier (110) inside the cavity (104) of the housing (102) of the electronic device (100);
   - emitting electromagnetic radiation from the emitter unit (112) into the cavity (104);
   - receiving at least part of the electromagnetic radiation from the cavity (104) with the detector unit (114) and generating a detector signal that represents the electromagnetic radiation received by the detector unit (114);
   - determining from the detector signal a volume of the liquid (M) inside the drug compartment (8, 81) using the processing unit (108);
   - detecting a presence of the identifier (110) from the detector signal using the processing unit (108); and
   - distinguishing, using the processing unit (108), the drug delivery device (10, 200) based on the identifier (110) from a further drug delivery device (10, 200) having a further identifier (110) that is different from the identifier (110),
   **characterlzed by**
   the identifier (110) being provided on, for example printed on or glued to, a surface (116) of the drug delivery device (10, 200).
2. The method according to embodiment 1, wherein the surface of the drug delivery device (10, 200) is selected from the group of members consisting of a surface of the cartridge (8), a surface of a dispensing unit (410), surface of a cartridge holder (2, 412), surface of a housing (3, 211),
3. The method according to embodiment 1 or embodiment 2, wherein the emitter unit (112) is selected from the group of members comprising one single emitter, a unit comprising several emitters and several emitters working as a unit.
4. The method according to one of the preceding embodiments, wherein the detector unit (114) is selected from the group of members one single detector, a detector unit comprising several individual detectors and several detectors working as a common detector unit.
5. Method according to one of the preceding embodiments, wherein the processing unit (108) determines the volume of the liquid (M) from a first signal component of the detector signal and detects the identifier (110) from a second signal component of the detector signal different from the first signal component,
   wherein, for example, the signal components amount to signals generated by spatially separated sensor elements of the detection unit (114).
6. Method according to one of the preceding embodiments,
   wherein the identifier (110) is located at a distal end (206) of the drug compartment (8, 81) facing away from a delivery end of the drug delivery device (10, 200).
7. Method according to one of the preceding embodiments, wherein the identifier (110) is located at a longitudinal position that radially overlaps with the drug compartment (8,81).
8. Method according to one of the preceding embodiments, wherein the identifier (110) comprises a first and second portion, wherein the first and second portion are spaced apart from each other along a longitudinal axis (207) of the drug delivery device (10, 200).
9. Method according to embodiment 8, wherein a distance between the first and second portion is larger than a longitudinal extent of a movable plunger (9) sealing the drug compartment (8, 81).
10. Method according to embodiment 8 or embodiment 9, wherein a distance between the first and second portions (110a, 110b) is one of constant and varying for different kinds of drug delivery devices (10, 200).
11. Method according to one of embodiments 8 to 10, wherein the first and second portions (110a, 110b) also differ in size and/or shape 0.
12. Method according to one of the preceding embodiments, wherein the identifier (110) comprises a pattern (118) with a modulated refractive index at a wavelength of the electromagnetic radiation.
13. Method according to embodiment 12, wherein the pattern (118) provides a modulated attenuation of the electromagnetic radiation, wherein the detector signal represents a shadow cast by the identifier (110) on the detector unit (114).
14. Method according to one of the preceding embodiments, wherein one of the identifier (110) and the pattern (118) comprises a circumferential marker (120) that surrounds a longitudinal axis of the drug delivery device (10, 200).
15. Method according to one of the preceding embodiments, wherein one of the identifier (110) and the pattern (118) comprises a longitudinal marker (122) that only covers a limited radial sector around a longitudinal axis of the drug delivery device (10, 200).
16. Method according to one of preceding embodiments, wherein the identifier is a coding feature for a specific a drug delivery device (10, 200), wherein the method comprises the step of decoding the coding feature to determine the type of a drug delivery device (10, 200) inserted into the cavity (104) of the electronic device (100).
17. Method according to embodiment 16, wherein the coding feature comprises a member selected from the group of members consisting of one or more straight lines of different size and/or colour, one or more curved lines of different size and/or colour, one or more zigzag lines of different size and/or colour, one or more dashed lines of different size and/or colour, fluorescent markings, a colored shape, a transparent code that filters only part of the spectrum of the electromagnet radiation; and combinations of the foregoing.
18. Method according to embodiment 16 or embodiment 17, wherein the coding feature comprises one or more dimensions of the identifier (110) and/or specifics, such as shape, of the identifier (110), with the one or more dimensions of the identifier (110) and/or specifics, such as shape, of the identifier (110) being detectable by the sensor assembly (106), i.e. the detection unit (114) that is also configured to detect a position of a plunger (9) present within the drug compartment (8, 81).
19. Method according to one of the preceding embodiments,
   wherein the identifier (110) is provided on a housing part of the housing (3, 211) surrounding the drug compartment (8, 81),
   wherein the housing part is transparent to the electromagnetic radiation.
20. Method according to one of the preceding embodiments,
   wherein the identifier (110) is provided at a cartridge holder (2, 412) of the drug delivery device (10, 200),
   wherein the cartridge holder (2, 412) is configured to receive a cartridge (8) that comprises the drug compartment (8, 81) or is integrally formed with the cartridge (8).
21. Method according to one of the preceding embodiments,
   wherein the drug compartment (8, 81) is sealed by a movable plunger (9),
   wherein the volume of the liquid (M) inside the drug compartment (8, 81) is determined by determining a longitudinal position of the plunger (9) within the drug compartment (8, 81).
22. Method according to one of the preceding embodiments, wherein the housing (102) of the electronic device (100) is configured as a removable cap (124) of the drug delivery device (10, 200), wherein the removable cap (124) is configured to surround a proximal delivery end of the drug delivery device (10, 200).
23. Method according to claim 20, wherein the cap is configured to be attachable to the drug delivery device (10, 200) in a single rotational position only.
24. Method according to one of the preceding embodiments,
   wherein the detector unit (114) is configured as a position resolving detector, such as a detector array comprising several longitudinally spaced-apart detector elements.
25. Method according to one of the preceding embodiments,
   wherein the emitting of the electromagnetic radiation into the cavity (104) and the receiving of the electromagnetic radiation from the cavity (104) is performed while the drug compartment (8, 81) and the identifier (110) are received stationary inside the cavity (104).
26. Method according to one of embodiments 1 to 24,
   wherein the emitting of the electromagnetic radiation into the cavity (104) and the receiving of the electromagnetic radiation from the cavity (104) is performed while the drug compartment (8, 81) and the identifier (110) are moved into and/or out of the cavity (104).
27. Method according to one of the preceding embodiments,
   wherein the electronic device (100) comprises a memory (126) that stores at least two different sets of configuration data, each set of configuration data being associated with a different type of drug delivery device (10, 200) and/or liquid (M), wherein the method further comprises:
   - selecting a selected set from the sets of configuration data using the processing unit (108) based on the identifier (110) detected by the processing unit (108).
28. Method according to embodiment 27, wherein the configuration data comprise calibration data, wherein the volume of the liquid (M) inside the drug compartment (8, 81) is determined by the processing unit (108) using the calibration data of the selected set.
29. Method according to one of the preceding embodiments,
   wherein the method further comprises:
   - comparing a type of the identified drug delivery device (10, 200) with a predetermined type,
   - generating a warning if the type of the identified drug delivery device (10, 200) deviates from the predetermined type.
30. Method according to one of the preceding embodiments,
   wherein the method further comprises:
   - receiving a foreign object having no identifier (110) inside the cavity (104) of the housing (102) of the electronic device (100);
   - generating a warning by the processing unit (108) upon a failed detection of an identifier (110) after receiving the foreign object.
31. Set of a drug delivery device (10, 200), such as an injection pen, and an electronic device (100) for attachment to the drug delivery device (10, 200),
   wherein the drug delivery device (10, 200) comprises a drug compartment (8, 81) for holding a liquid (M) to be delivered by the drug delivery device (10, 200) and an identifier (110) provided on a surface (116) of the drug delivery device (10, 200),
   wherein the electronic device (100) comprises a housing (102), a sensor assembly (106) and a processing unit (108),
   wherein the sensor assembly (106) and the processing unit (108) are arranged inside the housing (102),
   wherein the housing (102) comprises a cavity (104) that is configured to receive the drug compartment (8, 81) and the identifier (110) of the drug delivery device (10, 200),
   wherein the sensor assembly (106) comprises an emitter unit (112) and a detector unit (114),
   wherein the emitter unit (112) is configured to emit electromagnetic radiation into the cavity (104),
   wherein the detector unit (114) is configured to receive at least part of the electromagnetic radiation from the cavity (104) and to generate a detector signal that represents the electromagnetic radiation received by the detector unit (114),
   wherein the processing unit (108) is configured to determine from the detector signal a volume of the liquid (M) inside the drug compartment (8, 81),
   wherein the processing unit (108) is further configured to detect a presence of the identifier (110) from the detector signal and to distinguish the drug delivery device (10, 200) based on the identifier (110) from a further drug delivery device (10, 200) having a further identifier (110) that is different from the identifier (110).
32. Set according to embodiment 31, wherein the electronic device (100) is configured to carry out a method in accordance with embodiments 1 to 30.
33. Set according to embodiment 31 or embodiment 32, wherein the drug delivery device (10, 200) is one of a disposable drug delivery device (10) and a reusable drug delivery device (200).
34. Set according to one of embodiments 31 to 33, wherein the set further comprises one or more further drug delivery devices (10, 200).
35. Set according to embodiment 34, wherein the one or more further drug delivery devices (10, 200) are selected from the group of members consisting of: one or more disposable drug delivery devices (10), one or more reusable drug delivery devices (200) and combinations of disposable drug delivery devices (10) and reusable drug delivery devices (200).
36. Electronic device (100) for attachment to a drug delivery device (10, 200), such as an injection pen,
   wherein the electronic device comprises a housing (102), a sensor assembly (106) and a processing unit (108),
   wherein the sensor assembly (106) and the processing unit (108) are arranged inside the housing (102),
   wherein the housing (102) comprises a cavity (104) that is configured to receive a drug compartment (8, 81) for holding a liquid (M) to be delivered by the drug delivery device (10, 200) and an identifier (110) of the drug delivery device (10, 200) that is provided on a surface (116) of the drug delivery device (10, 200),
   wherein the sensor assembly (106) comprises an emitter unit (112) and a detector unit (114),
   wherein the emitter unit (112) is configured to emit electromagnetic radiation into the cavity (104),
   wherein the detector unit (114) is configured to receive at least part of the electromagnetic radiation from the cavity (104) and to generate a detector signal that represents the electromagnetic radiation received by the detector unit (114),
   wherein the processing unit (108) is configured to determine from the detector signal a volume of the liquid (M) inside the compartment,
   wherein the processing unit (108) is further configured to detect a presence of the identifier (110) from the detector signal and to distinguish the drug delivery device (10, 200) based on the identifier (110) from a further drug delivery device (10, 200) having a further identifier (110) that is different from the identifier (110).
37. Electronic device (100) according to embodiment 36, wherein the electronic device (100) is configured to carry out a method in accordance with embodiments 1 to 30.
38. A smart device configured to interact with the electronic device (100) in accordance with embodiment 36 and to carry out a method in accordance with embodiments 1 to 30.
39. A smart device in accordance with 38, wherein the smart device is configured to store measured amounts of medicament (M) associated with a kind of drug delivery device (10, 200) and to display said measured amounts of the medicament (M) in dependence on time and drug delivery device (10, 200).
40. Method for identifying a cartridge holder (2, 407), such as for a pen-injector, using an electronic device (100), in particular in accordance with a method of one of embodiments 1 to 30,
   wherein the electronic device (100) comprises a housing (102) having a cavity (104), a sensor assembly (106), and a processing unit (108),
   wherein the sensor assembly (106) and the processing unit (108) are arranged inside the housing (102),
   wherein the cartridge holder (2, 407) comprises a drug compartment (8, 81) for holding a liquid (M) to be delivered by a drug delivery device (10, 200) and an identifier (110),
   wherein the sensor assembly (106) comprises an emitter unit (112) and a detector unit (114),
   wherein the method comprises:
   - receiving the drug compartment (8, 81) and the identifier (110) inside the cavity (104) of the housing (102) of the electronic device (100);
   - emitting electromagnetic radiation from the emitter unit (112) into the cavity (104);
   - receiving at least part of the electromagnetic radiation from the cavity (104) with the detector unit (114) and generating a detector signal that represents the electromagnetic radiation received by the detector unit (114);
   - determining from the detector signal a volume of the liquid (M) inside the drug compartment (8, 81) using the processing unit (108);
   - detecting a presence of the identifier (110) from the detector signal using the processing unit (108); and
   - distinguishing, using the processing unit (108), the cartridge holder (2, 407) based on the identifier (110) from a further cartridge holder (2, 407) having a further identifier (110) that is different from the identifier (110),
   **characterlzed** by
   the identifier (110) being provided on, for example printed on or glued to, a surface (116) of the cartridge holder (2, 407).

## Claims

1. Method for identifying a drug delivery device (10, 200), such as a pen-injector, using an electronic device (100),
wherein the electronic device (100) comprises a housing (102) having a cavity (104), a sensor assembly (106), and a processing unit (108),
wherein the sensor assembly (106) and the processing unit (108) are arranged inside the housing (102),
wherein the drug delivery device (10, 200) comprises a drug compartment (8, 81) for holding a liquid (M) to be delivered by the drug delivery device (10, 200) and an identifier (110),
wherein the sensor assembly (106) comprises an emitter unit (112) and a detector unit (114),
wherein the method comprises:
- receiving the drug compartment (8, 81) and the identifier (110) inside the cavity (104) of the housing (102) of the electronic device (100);
- emitting electromagnetic radiation from the emitter unit (112) into the cavity (104);
- receiving at least part of the electromagnetic radiation from the cavity (104) with the detector unit (114) and generating a detector signal that represents the electromagnetic radiation received by the detector unit (114);
- determining from the detector signal a volume of the liquid (M) inside the drug compartment (8, 81) using the processing unit (108);
- detecting a presence of the identifier (110) from the detector signal using the processing unit (108); and
- distinguishing, using the processing unit (108), the drug delivery device (10, 200) based on the identifier (110) from a further drug delivery device (10, 200) having a further identifier (110) that is different from the identifier (110),
**characterlzed**by
the identifier (110) being provided on, for example printed on or glued to, a surface (116) of the drug delivery device (10, 200).

2. Method according to one of the preceding claims,
wherein the processing unit (108) determines the volume of the liquid (M) from a first signal component of the detector signal and detects the identifier (110) from a second signal component of the detector signal different from the first signal component, wherein, for example, the signal components amount to signals generated by spatially separated sensor elements of the detection unit (114).

3. Method according to one of the preceding claims, wherein the identifier (110) is located at a distal end (206) of the drug compartment (8, 81) facing away from a delivery end of the drug delivery device (10, 200).

4. Method according to one of the preceding claims,
wherein the identifier (110) is located at a longitudinal position that radially overlaps with the drug compartment (8, 81).

5. Method according to one of the preceding claims,
wherein the identifier (110) comprises a first and second portion,
wherein the first and second portion are spaced apart from each other along a longitudinal axis (207) of the drug delivery device (10, 200).

6. Method according to claim 0,
wherein a distance between the first and second portion is larger than a longitudinal extent of a movable plunger (9) sealing the drug compartment (8, 81).

7. Method according to one of the preceding claims,
wherein the identifier (110) comprises a pattern (118) with a modulated refractive index at a wavelength of the electromagnetic radiation.

8. Method according to one of the preceding claims,
wherein the pattern (118) provides a modulated attenuation of the electromagnetic radiation,
wherein the detector signal represents a shadow cast by the identifier (110) on the detector unit (114).

9. Method according to one of the preceding claims,
wherein one of the identifier (110) and the pattern (118) comprises a circumferential marker (120) that surrounds a longitudinal axis of the drug delivery device (10, 200).

10. Method according to one of the preceding claims,
wherein one of the identifier (110) and the pattern (118) comprises a longitudinal marker (122) that only covers a limited radial sector around a longitudinal axis of the drug delivery device (10, 200).

11. Method according to one of preceding claims,
wherein the identifier is a coding feature for a specific a drug delivery device (10, 200), wherein the method comprises the step of decoding the coding feature to determine the type of a drug delivery device (10, 200) inserted into the cavity (104) of the electronic device (100).

12. Method according to claim 11, wherein the coding feature comprises a member selected from the group of members consisting of one or more straight lines of different size and/or colour, one or more curved lines of different size and/or colour, one or more zigzag lines of different size and/or colour, one or more dashed lines of different size and/or colour, fluorescent markings, a colored shape, a transparent code that filters only part of the spectrum of the electromagnet radiation; and combinations of the foregoing.

13. Method according to one of the preceding claims,
wherein the identifier (110) is provided at a cartridge holder (2, 412) of the drug delivery device (10, 200),
wherein the cartridge holder (2, 412) is configured to receive a cartridge (8) that comprises the drug compartment (8, 81) or is integrally formed with the cartridge (8).

14. Method according to one of the preceding claims,
wherein the electronic device (100) comprises a memory (126) that stores at least two different sets of configuration data, each set of configuration data being associated with a different type of drug delivery device (10, 200) and/or liquid (M), wherein the method further comprises:
- selecting a selected set from the sets of configuration data using the processing unit (108) based on the identifier (110) detected by the processing unit (108).

15. Set of a drug delivery device (10, 200), such as an injection pen, and an electronic device (100) for attachment to the drug delivery device (10, 200),
wherein the drug delivery device (10, 200) comprises a drug compartment (8, 81) for holding a liquid (M) to be delivered by the drug delivery device (10, 200) and an identifier (110) provided on a surface (116) of the drug delivery device (10, 200), wherein the electronic device (100) comprises a housing (102), a sensor assembly (106) and a processing unit (108),
wherein the sensor assembly (106) and the processing unit (108) are arranged inside the housing (102),
wherein the housing (102) comprises a cavity (104) that is configured to receive the drug compartment (8, 81) and the identifier (110) of the drug delivery device (10, 200),
wherein the sensor assembly (106) comprises an emitter unit (112) and a detector unit (114),
wherein the emitter unit (112) is configured to emit electromagnetic radiation into the cavity (104),
wherein the detector unit (114) is configured to receive at least part of the electromagnetic radiation from the cavity (104) and to generate a detector signal that represents the electromagnetic radiation received by the detector unit (114),
wherein the processing unit (108) is configured to determine from the detector signal a volume of the liquid (M) inside the drug compartment (8, 81),
wherein the processing unit (108) is further configured to detect a presence of the identifier (110) from the detector signal and to distinguish the drug delivery device (10, 200) based on the identifier (110) from a further drug delivery device (10, 200) having a further identifier (110) that is different from the identifier (110).

16. Electronic device (100) for attachment to a drug delivery device (10, 200), such as an injection pen,
wherein the electronic device comprises a housing (102), a sensor assembly (106) and a processing unit (108),
wherein the sensor assembly (106) and the processing unit (108) are arranged inside the housing (102),
wherein the housing (102) comprises a cavity (104) that is configured to receive a drug compartment (8, 81) for holding a liquid (M) to be delivered by the drug delivery device (10, 200) and an identifier (110) of the drug delivery device (10, 200) that is provided on a surface (116) of the drug delivery device (10, 200),
wherein the sensor assembly (106) comprises an emitter unit (112) and a detector unit (114),
wherein the emitter unit (112) is configured to emit electromagnetic radiation into the cavity (104),
wherein the detector unit (114) is configured to receive at least part of the electromagnetic radiation from the cavity (104) and to generate a detector signal that represents the electromagnetic radiation received by the detector unit (114),
wherein the processing unit (108) is configured to determine from the detector signal a volume of the liquid (M) inside the compartment,
wherein the processing unit (108) is further configured to detect a presence of the identifier (110) from the detector signal and to distinguish the drug delivery device (10, 200) based on the identifier (110) from a further drug delivery device (10, 200) having a further identifier (110) that is different from the identifier (110).
